# EUROPÄISCHE PATENTSCHRIFT

(11) **EP 0 809 488 B1**
(45) Veröffentlichungstag und Bekanntmachung des Hinweises auf die Patenterteilung: **17.07.2002**
(21) Anmeldenummer: 96903958.5
(22) Anmeldetag: 01.02.1996
(51) Int. Cl.: A61K 9/22

(54) **FESTE WIRKSTOFF-ZUBEREITUNGEN**
SOLID ACTIVE AGENT PREPARATIONS
PREPARATIONS SOLIDES DE CONSTITUANTS ACTIFS

(30) Priorität: 14.02.1995 DE 19504832
(43) Veröffentlichungstag der Anmeldung: 03.12.1997
(73) Patentinhaber: BASF AKTIENGESELLSCHAFT, 67056 Ludwigshafen (DE)
(72) Erfinder: GRABOWSKI, Sven, D-67061 Ludwigshafen (DE); BREITENBACH, Jörg, D-68199 Mannheim (DE); ROSENBERG, Joerg, D-67158 Ellerstadt (DE); SANNER, Axel, D-67227 Frankenthal (DE)
(86) Internationale Anmeldenummer: EP9600417
(87) Internationale Veröffentlichungsnummer: WO9625151

(56) Entgegenhaltungen:
- EP-A- 0 398 033
- EP-A- 0 544 144
- CHEMICAL & PHARMACEUTICAL BULLETIN, Bd. 42, Nr. 9, September 1994, TOKYO (JP), Seiten 1902-1908, XP000476614 M. MATSUMURA ET AL.: "COMPUTER OPTIMIZATION FOR THE FORMULATION OF CONTROLLED-RELEASE THEOPHYLLINE TABLET MADE OF MICRONIZED LOW-SUBSTITUTED HYDROXYPROPYLCELLULOSE AND METHYLCELLULOSE"

## Beschreibung

Die vorliegende Erfindung betrifft feste Zubereitungen, erhältlich durch gemeinsame Schmelzextrusion von
A) einem oder mehreren Wirkstoffen,
B) einer Mischung aus
   B1) 10 bis 90 Gew.-% mindestens eines thermoplastisch verarbeitbaren, wasserlöslichen Polymers, und
   B2) 10 bis 90 Gew.-% einer niedrig substituierten wasserunlöslichen Hydroxypropylcellulose, und
C) 0 bis 50 Gew.-%, bezogen auf die Gesamtmenge der Zubereitung, eines oder mehrerer pharmazeutischer Hilfsstoffe.

Weiterhin betrifft die Erfindung ein Verfahren zur Herstellung solcher Zubereitungen sowie deren Verwendung als Arzneimittel.

Wirkstoffhaltige Zubereitungen, die durch Schmelzextrusion hergestellt werden, sind allgemein bekannt.

Das Extrudieren von wirkstoffhaltigen Schmelzen wasserlöslicher Polymeren, vorzugsweise von Copolymeren des Vinylpyrrolidons, ist in der EP-A 240 904 und der EP-A 240 906 beschrieben.

Aus der JP-A 58-192817 und der JP-A 58-79915 ist die Schmelzextrusion von wirkstoffhaltigen Zubereitungen auf Basis von thermoplastischen Polymeren wie beispielsweise Hydroxypropylcellulose als Bindemittel bekannt.

Niedrig substituierte Hydroxypropylcellulose (L-HPC), die durch partielle Veretherung der Cellulose mit Propylenoxid hergestellt wird, ist in Wasser unlöslich, quillt aber bei Kontakt mit Wasser. Aufgrund dieses Quellverhaltens wird L-HPC als Sprengmittel zur Zerfallsbeschleunigung von Tabletten eingesetzt. L-HPC kann auch als Bindemittel für Tabletten zur Erhöhung der Tablettenhärte eingesetzt werden.

Kawashima et al., Chem. Pharm. Bull. 41 (1933), 1827-31, beschreiben, daß bei Einsatz von L-HPC in Granulaten für die Tablettierung zum einen stark von der Partikelgröße der L-HPC beeinflußt werden, zum anderen das Wirkstofffreisetzungsprofil entscheidend durch die Preßkraft bei der Kompaktierung beeinflußt wird.

Im Gegensatz zu Hydroxypropylcellulosen mit höheren Substitutionsgraden zeigt die L-HPC jedoch keine thermoplastische Verarbeitbarkeit.

Aufgabe der vorliegenden Erfindung war es, Wirkstoff-Zubereitungen zu finden, welche durch Polymer-Wirkstoff-Schmelzextrusion hergestellt werden können und eine gezielte Einstellung der Wirkstofffreisetzung erlauben.

Demgemäß wurden die eingangs definierten Zubereitungen, ein Verfahren zu deren Herstellung sowie deren Verwendung als Arzneimittel gefunden.

Als Komponente A) kommen solche Wirkstoffe in Betracht, die sich unter den Verarbeitungsbedingungen bei der Schmelzextrusion nicht zersetzen.

### Geeignete Wirkstoffe sind beispielsweise:

Acebutolol, Acetylcysteine, Acetylsalicylsäure, Aciclovir, Alprazolam, Albumin, Alfacalcidol, Allantoin, Allopurinol, Ambroxol, Amikacin, Amiloride, Aminoessigsäure, Amiodarone, Amitriptyline, Amlodipine, Amoxicillin, Ampicillin, Ascorbic Acid, Aspartam, Astemizole, Atenolol, Beclometasone, Benserazide, Benzalkonium Hydroxid, Benzocaine, Benzoesäure, Betametasone, Bezafibrate, Biotin, Biperiden, Bisoprolol, Bromazepam, Bromhexine, Bromocriptine, Budesonide, Bufexamac, Buflomedil, Buspirone, Coffein, Campher, Captopril, Carbamazepine, Carbidopa, Carboplatin, Cefachlor, Cefalexin, Cefadroxil, Cefazolin, Cefixime, Cefotaxime, Ceftazidine, Ceftriaxone, Cefuroxime Axetil, Chloramphenicol, Chlorhexidine, Chlorpheniramine, Chlortalidone, Choline, Ciclosporin, Cilastatin, Cimetidine, Ciprofloxacin, Cisapride, Cisplatin, Clarithromycin, Clavulansäure, Clomibramine, Clonazepam, Clonidine, Clotrimazole, Clozapin, Codeine, Colestyramine, Cromoglicinsäure, Cyanocobalamin, Cyproterone, Desogestrel, Dexamethasone, Dexpanthenol, Dexthromethorphan, Dextropropoxiphene, Diazepam, Diclofenac, Digoxin, Dihydrocodeine, Dihydroergotamine, Diltiazem, Diphenhydramine, Dipyridamole, Dipyrone, Disopyramide, Domperidone, Dopamine, Enalapril, Ephedrine, Epinephrine, Ergocalciferol, Ergotamine, Erythromycin, Estradiol, Ethinylestradiol, Etoposide, Eucalyptus Globulus, Famotidine, Felodipine, Fenofibrate, Fenoterol, Fentanyl, Flavin Mononucleotide, Fluconazole, Flunarizine, Fluorouracil, Fluoxetine, Flurbiprofen, Furosemide, Gemfibrozil, Gentamicin, Ginkgo Biloba, Glibenclamide, Glipizide, Glycyrrhiza Glabra, Guaifenesin, Haloperidol, Heparin, Hyaluronsäure, Hydrochlorothiazide, Hydrocodone, Hydrocortisone, Hydromorphon, Ipratropium Hydroxide, Ibuprofen, Imipenem, Indomethacin, Iohexol, Iopamidol, Isosorbide Dinitrate, Isosorbide Mononitrate, Isotretinoin, Ketotifen, Ketoconazole, Ketoprofen, Ketorolac, Labetalol, Lactulose, Lecithin, Levocarnitine, Levodopa, Levoglutamide, Levonorgestrel, Levothyroxine, Lidocaine, Lipase, Lisinopril, Loperamide, Lorazepam, Lovastatin, Medroxyprogesterone, Menthol, Methotrexate, Methyldopa, Methylprednisolone, Metoclopramide, Metoprolol, Miconazole, Midazolam, Minocycline, Minoxidil, Misoprostol, Morphine, Multivitamine und Mineralien, Nystatin, N-Methylephedrine, Naftidrofuril, Naproxen, Neomycin, Nicardipine, Nicergoline, Nicotinamide, Nicotine, Nicotinsäure, Nifedipine, Nimodipine, Nitrendipine, Nizatidine, Norethisterone, Norfloxacin, Norgestrel, Nortriptyline, Ofloxacin, Omeprazole, Ondansetron, Pancreatin, Panthenol, Pantothensäure, Paracetamol, Penicillin G, Penicillin V, Phenobarbital, Pentoxifylline, Phenylephrine, Phenylpropanolamin, Phenytoin, Piroxicam, Polymyxin B, Povidone-Iod, Pravastatin, Prazosin, Prednisolone, Propafenone, Propranolol, Pseudoephedrine, Pyridoxine, Quinidine, Ramipril, Ranitidine, Reserpine, Retinol, Riboflavin, Rifampicin, Rutoside, Saccharin, Salbutamol, Salcatonin, Salicylsäure, Selegilin, Simvastatin, Somatropin, Sotalol, Spironolactone, Sucralfate, Sulbactam, Sulfamethoxazole, Sulpiride, Tamoxifen, Tegafur, Teprenone, Terazosin, Terbutaline, Terfenadine, Theophylline, Thiamine, Ticlopidine, Timolol, Tranexamsäure, Tretinoin, Triamcinolone Acetonide, Triamterene, Trimethoprim, Troxerutin, Uracil, Valproinsäure, Vancomycin, Verapamil, Vitamin E, Volinic Acid, Zidovudine.

Weiterhin kommen als Wirkstoffe auch Vitamine wie beispielsweise Vitamin C, β-Carotin und andere Carotinoide oder Pflanzenschutzmittel in Betracht.

Bevorzugt liegen die Wirkstoffe in Form sogenannter "fester Lösungen", d.h. molekulardispers in der Matrix verteilt, oder in Form einer festen Dispersion vor.

Die Menge der Wirkstoffkomponente A) in der Gesamtzubereitung kann je nach Wirksamkeit und Freisetzungsgeschwindigkeit in weiten Grenzen variieren. So kann der Wirkstoffgehalt im Bereich von 0,1 bis 90 Gew.-%, vorzugsweise von 0,5 bis 60 Gew.-%, bezogen auf die gesamte Zubereitung, liegen. Die einzige Bedingung ist, daß die Zubereitung noch thermoplastisch verarbeitbar ist.

Als polymere Komponenten B) enthalten die erfindungsgemäßen Zubereitungen eine Mischung aus
B1) 10 bis 90 Gew.-%, vorzugsweise 20 bis 80 Gew.-% eines wasserlöslichen, thermoplastischen Polymeren, und
B2) 10 bis 90 Gew.-%, vorzugsweise 20 bis 80 Gew.-% einer wasserunlöslichen niedrig substituierten Hydroxypropylcellulose,
wobei sich die Mengenangaben auf die Summe der Mengen an B1) und B2) beziehen.

Als wasserlösliche Polymere B1) seien genannt:
- Alkylcellulosen wie Methylcellulose,
- Hydroxyalkylcellulosen wie Hydroxymethyl-, Hydroxyethyl-, Hydroxypropyl- und Hydroxybutylcellulose,
- Hydroxyalkylalkylcellulosen wie Hydroxyethylmethyl- und Hydroxypropylmethylcellulose,
- Polyvinylpyrrolidon,
- Copolymere aus N-Vinylpyrrolidon und Vinylacetat mit bis zu 50 Gew.-% Vinylacetat,
- Carboxyalkylcellulosen wie Carboxymethylcellulosen,
- Polysaccharide wie Alginsäure und deren Alkali- und Ammoniumsalze,
sowie Gemische solcher wasserlöslichen Polymeren.

Die Komponente B1) soll in der Gesamtmischung aller Komponenten im Bereich von 50 bis 180°C, vorzugsweise 60 bis 150°C erweichen oder schmelzen, so daß die Masse extrudierbar ist. Die Glasübergangstemperatur der Polymeren soll demgemäß unter 180°C liegen.

"Wasserlöslich" bedeutet, daß sich in 100 g Wasser von 20°C mindestens 0,5 g, vorzugsweise mindestens 2 g des Polymeren lösen, gegebenenfalls auch kolloidal.

Bevorzugt wird als Polymerkomponente A) Hydroxypropylcellulose mit einem molaren Substitutionsgrad von 3,0 bis 4,4 verwendet.

Komponente B2) ist erfindungsgemäß eine niedrig substituierte Hydroxypropylcellulose mit einem molaren Substitutionsgrad von 0,5 bis 2, bevorzugt 1,5 bis 1,8, die sogenannte low-substituted hydroxypropylcellulose (L-HPC) wie sie in der US-Pharmakopöe/NF XVII und der japanischen Pharmakopöe JP XI beschrieben ist. Solche L-HPC ist wasserunlöslich, aber wasserquellbar und verhält sich nicht thermoplastisch.

Innerhalb der angegebenen Grenzen richtet sich die Menge an eingesetzter Komponente B2) vorzugsweise danach, welche Wirkstofffreisetzungsgeschwindigkeit gewünscht wird. Für den Fall schneller Freisetzung empfiehlt sich der Einsatz geringerer Mengen, beispielsweise 5 bis 30 Gew.-%. Für den Fall, daß eine verzögerte Wirkstofffreisetzung gewünscht ist, empfiehlt sich der Einsatz von 30 bis 90 Gew.-% an B2).

Der Korngröße der eingesetzten L-HPC ist erfindungsgemäß nicht kritisch.

Als Komponenten C) können die erfindungsgemäßen Zubereitungen die üblichen pharmazeutischen Hilfsstoffe wie Füllstoffe, Schmiermittel, Formentrennmittel, Fließregulierungsmittel, Weichmacher, Farbstoffe und Stabilisatoren in Mengen bis zu ca. 50 Gew.-% enthalten. Diese und die im folgenden angegebenen Mengen sind jeweils bezogen auf das Gesamtgewicht der Zubereitung (= 100 %).

Als Füllstoffe seien z.B. die Oxide von Magnesium, Aluminium, Silicium und Titan sowie Lactose, Mannit, Sorbit, Xylit, Pentaerythrit und seine Derivate genannt, wobei die Menge an Füllstoff bei ca. 0,02 bis 50, vorzugsweise 0,2 bis 20 Gew.-% liegt.

Als Fließregulierungsmittel seien z.B. die Mono-, Di- und Triglyceride der langkettigen Fettsäuren wie C₁₂-, C₁₄-, C₁₆- und C₁₈-Fettsäure, Wachse wie Carnaubawachs sowie die Lecithine genannt, wobei die Menge bei ca. 0,1 bis 30, vorzugsweise 0,1 bis 5 Gew.-% liegt.

Als Weichmacher seien z.B. neben niedermolekularen Polyalkylenoxiden wie Polyethylenglykol, Polypropylenglykol und Polyethylenpropylenglykol auch mehrwertige Alkohole wie Propylenglykol, Glycerin, Pentaerythrit und Sorbit sowie Natriumdiethylsulfosuccinat, Mono-, Di- und Triacetat des Glycerin und Polyethylenglykolstearinsäureester genannt. Dabei liegt die Menge an Weichmacher bei ca. 0,5 bis 15, vorzugsweise 0,5 bis 5 Gew.-%.

Als Schmiermittel seien z.B. Stearate von Aluminium oder Calcium sowie Talkum und Silikone genannt, wobei ihre Menge bei ca. 0,1 bis 5, vorzugsweise 0,1 bis 3 Gew.-% liegt.

Als Stabilisatoren seien beispielsweise Lichtstabilisatoren, Antioxidantien, Radikalfänger und Stabilisatoren gegen mikrobiellen Befall genannt, wobei ihre Menge vorzugsweise bei ca. 0,01 bis 0,05 Gew.-% liegt.

Um die erfindungsgemäßen Zubereitungen herzustellen, kann die Wirkstoffkomponente entweder direkt in Form einer physikalischen Mischung mit den Polymeren B verschmolzen werden oder mit der bereits vorliegenden Polymerschmelze gemischt werden.

Im übrigen erfolgt die Vermischung der Komponente A) mit der Schmelze in an sich bekannter Weise in Extrudern, vorzugsweise in Ein- oder Doppelschneckenextrudern in einem Temperaturbereich zwischen 50 und 200°C. Die Formgebung der wirkstoffhaltigen Polymerschmelze zu den erfindungsgemäßen Zubereitungen kann beispielsweise durch Kalandrierung des Extrudates nach der in der EP-A 240 906 beschriebenen Methode sowie nach dem aus der DE-A 38 30 355 bekannten Verarbeitungsverfahren durch Zerkleinerung des Extrudates mit rotierenden Messern in volumengleiche - noch verformbare - Stücke mit erstarrter Oberfläche und anschließendes Verpressen zu Tabletten in den üblichen Tablettiermaschinen erfolgen.

Es ist möglich, die Hilfsstoffe in die Schmelze oder Lösung aus Wirkstoffen und Polymeren B zu mischen. Ferner können die Hilfsstoffe zusammen mit dem Wirkstoff in die Polymerschmelze eingearbeitet werden. Außerdem können Gemische aus Hilfsstoffen, dem Wirkstoff und den Polymeren B direkt verschmolzen werden. Im allgemeinen ist es üblich, eine physikalische Mischung aus Hilfsstoffen, Wirkstoffen und den Polymeren B gemeinsam zu verschmelzen.

Die erfindungsgemäßen Zubereitungen werden als Arzneimittel verwendet und in Form von Tabletten, Pellets, Granulaten oder Kapseln eingesetzt. Vorzugsweise werden mit dem erfindungsgemäßen Zubereitungen Arzneiformen mit verzögerter Wirkstofffreisetzung hergestellt.

Falls gewünscht, kann die feste pharmazeutische Form auch mit einem üblichen Überzug zur Verbesserung des Aussehens und/oder des Geschmacks (Dragee) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt.

Die vorliegende Erfindung ermöglicht auf einfache Weise eine gezielte Einstellung des Wirkstofffreisetzungsprofils der erfindungsgemäßen festen Arzneiformen, vor allem bei der Herstellung von festen Arzneiformen mit verzögerter Wirkstofffreisetzung. Überraschenderweise gelingt dies unabhängig von der Partikelgröße der L-HPC und Verfahrensparametern bei der Formgebung.

### Beispiele 1 bis 3

Die in der Tabelle angegebenen Mengen an Wirkstoff und den Polymeren B1) und B2) wurden gemischt, in einen Doppelschnecken-extruder (ZSK 30, Firma Werner & Pfleiderer) eingebracht und über 5 Temperaturzonen extrudiert. Die Temperaturen der einzelnen Temperaturzonen ("Schüsse" 1-5) sind jeweils in der Tabelle I angegeben. Die über die Extruderdüsenleiste austretenden Schmelzstränge wurden durch luftgekühlten Heißabschlag mit einem Messerwalzengranulator pelletiert.

Die Wirkstofffreisetzung wurde mittels der Rührflügelmethode (paddle-Methode nach USP XXI, US-Arzneibuch) gemessen. Diese invitro-Methode dient zur Bestimmung der Lösungsrate von wirkstoffhaltigen Formlingen (z.B. Tabletten, Pellets etc.).

Hierzu wurden 900 ml eines Phosphatpuffers mit einem pH-Wert von 6,8 mit 0,1 Gew.-% Natriumlaurylsulfat in einem 1 1-Gefäß mit Rundboden auf 37°C temperiert und 300 g Pellets der Korngröße 1,25 bis 1,60 mm hinzugegeben. Die Wirkstofffreisetzung der Pellets wurde bei einer paddle-Drehzahl von 100 Upm nach jeweils 1, 2, 3, 4, 5, 6, 7 und 8 Stunden UV-spektroskopisch bestimmt.

Die Ergebnisse dieses Tests sind in Tabelle II aufgeführt.

**Tabelle I**

| Beispiel | Nifedipin Gew.-% | Polymer B1)¹⁾ Gew.-% | Polymer B2)²⁾ Gew.-% | Temperatur Schüsse 1-5 |
|---|---|---|---|---|
| 1 | 20 | 50 | 30 | 70,120,110, 100,100 |
| 2 | 20 | 40 | 40 | 60,120, 120,110,120 |
| 3 | 20 | 30 | 50 | 60,120,120, 120,130 |

| | | | | |
|---|---|---|---|---|
| 1) Hydroxypropylcellulose mit einem molaren Substitutionsgrad von 3,0 bis 4,4 (Klucel EF, Firma Hercules, USA) | | | | |
| 2) Hydroxypropylcellulose mit einem molaren Substitutionsgrad von 1,5 bis 1,8 (LH 31, Firma Shin-Etsu Chemical Comp. Ltd., Japan) | | | | |

**Tabelle II**

| Beispiel | Freisetzung in % nach Stunden | | | | | | | |
|---|---|---|---|---|---|---|---|---|
| | 1 | 2 | 3 | 4 | 5 | 6 | 7 | 8 |
| 1 | 32 | 70 | 91 | 99 | 100 | 100 | 100 | 100 |
| 2 | 31 | 60 | 77 | 89 | 96 | 100 | 100 | 100 |
| 3 | 24 | 43 | 57 | 69 | 75 | 81 | 87 | 91 |

## Patentansprüche

1. Feste Zubereitungen, erhältlich durch gemeinsame Schmelzextrusion von
A) einem oder mehreren Wirkstoffen,
B) einer Mischung aus
B1) 10 bis 90 Gew.-% mindestens eines thermoplastisch verarbeitbaren, wasserlöslichen Polymers, und
B2) 10 bis 90 Gew.-% einer niedrig substituierten wasserunlöslichen Hydroxypropylcellulose, und
C) 0 bis 50 Gew.-%, bezogen auf die Gesamtmenge der Zubereitung, eines oder mehrerer pharmazeutischer Hilfsstoffe.

2. Zubereitungen nach Anspruch 1, enthaltend als Komponente B2) eine Hydroxypropylcellulose mit einem molaren Substitutionsgrad von 1,5 bis 1,8.

3. Zubereitungen nach Anspruch 1 oder 2, enthaltend als Komponente B1) eine wasserlösliche Hydroxypropylcellulose.

4. Verfahren zur Herstellung der Zubereitungen gemäß den Ansprüchen 1 bis 3, **dadurch gekennzeichnet, daß** man die Wirkstoffkomponente A) mit der polymeren Komponente B) und gegebenenfalls die Hilfsstoffe C) zu einer Schmelze verarbeitet, die Schmelze extrudiert und unter Formgebung weiterverarbeitet.

5. Feste Arzneiformen aus den Zubereitungen gemäß den Ansprüchen 1 bis 3.

## Claims

1. A solid preparation, obtainable by joint melt extrusion of
A) one or more active compounds,
B) a mixture of
B1) from 10 to 90% by weight of at least one thermoplastically processable, water-soluble polymer, and
B2) from 10 to 90% by weight of a low-substituted water-insoluble hydroxypropylcellulose, and
C) from 0 to 50% by weight, based on the total amount of the preparation, of one or more pharmaceutical auxiliaries.

2. A preparation as claimed in claim 1, comprising as component B2) a hydroxypropylcellulose having a degree of molar substitution of from 1.5 to 1.8.

3. A preparation as claimed in claim 1 or 2, comprising as component B1) a water-soluble hydroxypropylcellulose.

4. A process for producing the preparations as claimed in claims 1 to 3, which comprises processing the active compound component A) with the polymeric component B) and, if appropriate, the auxiliaries C) to give a melt, extruding the melt and further processing with shaping.

5. A solid pharmaceutical form made from the preparations as claimed in claims 1 to 3.

## Revendications

1. Compositions solides obtenues par extrusion en commun à l'état fondu de
A) une ou plusieurs substances actives,
B) un mélange de
B1) 10 à 90 % en poids d'au moins un polymère soluble dans l'eau et apte au travail thermoplastique, et
B2) 10 à 90 % en poids d'une hydroxypropylcellulose à bas degré de substitution, insoluble dans l'eau, et
C) 0 à 50 % en poids, par rapport à la quantité totale de la composition, d'un ou plusieurs produits auxiliaires pharmaceutiques.

2. Compositions selon la revendication 1, contenant en tant que composant B2) une hydroxypropylcellulose au degré de substitution molaire de 1,5 à 1,8.

3. Compositions selon la revendication 1 ou 2, contenant en tant que composant B1) une hydroxypropylcellulose soluble dans l'eau.

4. Procédé pour la préparation des compositions selon les revendications 1 à 3, **caractérisé par le fait que** l'on combine le composant actif A) avec le composant polymère B) et le cas échéant les produits auxiliaires C) à l'état fondu, on extrude la masse fondue et on poursuit par un façonnage.

5. Formes médicamenteuses solides consistant en les compositions selon les revendications 1 à 3.
